# FASCICULE DE BREVET EUROPEEN

(11) **EP 3 145 576 B1**
(45) Date de publication et mention de la délivrance du brevet: **25.03.2020**
(21) Numéro de dépôt: 15725893.0
(22) Date de dépôt: 07.05.2015
(51) Int. Cl.: A61N 1/04, A61N 1/36

(54) **DISPOSITIF CUTANE, NOTAMMENT GENERATEUR D'IMPULSIONS POUR L'ELECTROSTIMULATION**
KUTANE VORRICHTUNG, INSBESONDERE EIN IMPULSGENERATOR ZUR ELEKTRISCHEN STIMULATION
CUTANEOUS DEVICE, IN PARTICULAR A PULSE GENERATOR FOR ELECTRICAL STIMULATION

(30) Priorité: 19.05.2014 FR 1454466
(43) Date de publication de la demande: 29.03.2017
(73) Titulaire: Commissariat à l'Énergie Atomique et aux Énergies Alternatives, 75015 Paris (FR)
(72) Inventeur: PERRAUD, Simon, 83150 Bandol (FR); KARST, Nicolas, 57600 Folkling (FR); SALOMON, Jérémie, 38250 Villard-de-Lans (FR)
(74) Mandataire: Gevers & Orès
(86) Numéro de dépôt international: PCT/IB2015/053340
(87) Numéro de publication internationale: WO 2015/177670

(56) Documents cités:
- US-A1- 2009 076 345
- US-A1- 2012 232 634
- US-A1- 2013 023 816
- US-A1- 2013 197 341

## Description

L'invention concerne le domaine technique des dispositifs destinés à être fixés sur la peau d'un utilisateur.

Il s'agit notamment de dispositifs médicaux, comme des électrodes cutanées pour la mesure de paramètres physiologiques ou des générateurs d'impulsions pour l'électrostimulation.

On rappelle ici que l'électrostimulation est une technique consistant à stimuler électriquement les nerfs ou les muscles. L'électrostimulation des nerfs (notamment la technique d'électrostimulation transcutanée des nerfs, ou transcutaneous electrical nerve stimulation ou TENS dans la terminologie anglaise) permet de traiter la douleur. L'électrostimulation des muscles (neuromuscular electrical stimulation ou NMES dans la terminologie anglaise) est utilisée à des fins de rééducation ou de musculation.

Les dispositifs d'électrostimulation comprennent un générateur d'impulsions électriques relié à des électrodes cutanées. Le générateur d'impulsions se présente sous la forme d'un boitier volumineux et rigide et permet d'envoyer des impulsions électriques calibrées en fréquence et en intensité jusqu'à une zone précise du corps humain par l'intermédiaire des électrodes.

A titre d'exemple, un générateur d'impulsions tel que décrit dans le document Mark Johnson, Transcutaneous Electrical Nerve Stimulation (Johnson, Mark I (October 2012), Transcutaneous Electrical Nerve Stimulation (TENS), eLS. John Wiley & Sons, Ltd: Chichester) se présente sous la forme d'un boitier de volume de l'ordre de plusieurs dizaines de centimètres cube et d'épaisseur de l'ordre de quelques centimètres.

Ce type de générateur d'impulsions n'est pas pratique d'utilisation. En effet, il s'agit d'un objet encombrant qui est relié aux électrodes cutanées par l'intermédiaire de câbles électriques relativement longs.

Il a été proposé dans l'art antérieur de remplacer les générateurs d'impulsions conventionnels, volumineux et rigides, par des générateurs d'impulsions fins et flexibles qui peuvent être directement portés par l'utilisateur sous la forme de patchs.

Un tel générateur d'impulsions fin et flexible, se présentant sous la forme d'un patch, est décrit dans le document US-5,423,874.

Dans ce patch, sont assemblés, sur un circuit flexible, la source d'énergie constituée d'une batterie et les composants électroniques qui sont des composants discrets et des circuits intégrés permettant de faire fonctionner le générateur d'impulsions.

Le circuit flexible est encapsulé entre une couche supérieure imperméable et une couche inférieure adhésive. La couche supérieure imperméable joue le rôle de barrière à l'humidité pour protéger la source d'énergie et les composants électroniques. La couche inférieure adhésive, qui comprend deux électrodes, permet de fixer le générateur d'impulsions sur la peau.

Ce générateur d'impulsions est moins encombrant que les générateurs d'impulsions conventionnels et il permet de réduire considérablement la longueur des câbles électriques, voire de se passer complètement de ces câbles.

Cependant, un dispositif tel que décrit dans le document US-5,423,874 n'offre pas une excellente fiabilité. En effet, étant donné que les couches d'encapsulation supérieure et inférieure sont des couches fines et flexibles, les composants du dispositif ne bénéficient pas d'une protection mécanique efficace, notamment contre les chocs, le percement ou les contraintes lors de la mise en flexion du dispositif.

Un dispositif destiné à être fixé sur la peau est décrit dans le document WO2009/036313A1.

L'invention a pour objet de pallier ces inconvénients en proposant un dispositif destiné à être fixé sur la peau d'un utilisateur qui soit à la fois fin, flexible et robuste.

Ce dispositif peut notamment constituer un générateur d'impulsions pour l'électrostimulation se présentant sous la forme d'un patch.

Ce dispositif peut trouver d'autres applications, notamment dans des dispositifs électroniques portables ou encore des dispositifs électroniques intégrés dans un textile.

Ainsi, l'invention concerne un dispositif destiné à être fixé sur la peau d'un utilisateur, sensiblement plan et comprenant des composants ainsi que des moyens de connexion électrique définissant au moins une zone rigide du dispositif, les moyens de connexion électrique présentant, dans le plan du dispositif, une surface correspondant à au moins un composant et étant disposés de manière à recouvrir ledit au moins un composant pour assurer sa protection mécanique.

Ainsi, le fait que les moyens de connexion électrique assurent également une fonction de protection mécanique permet d'assurer la fiabilité et la robustesse du dispositif cutané tout en améliorant sa compacité et en diminuant son épaisseur.

De façon préférée, les moyens de connexion électrique définissent au moins deux zones rigides reliées électriquement et mécaniquement par une zone flexible, lesdites zones rigides portant des composants.

Les moyens de connexion présentent avantageusement, dans le plan du dispositif, une surface supérieure à celle occupée par lesdits composants et sont alignés avec lesdits composants selon l'épaisseur d'au moins une zone rigide, de manière à les recouvrir.

De préférence, lesdits moyens de connexion électrique comportent au moins un trou destiné à coopérer avec une protubérance d'une fiche reliée à un conducteur électrique.

Lesdits moyens de connexion électrique remplissent avantageusement aussi une fonction magnétique, de façon à faciliter la connexion entre les moyens de connexion électrique du dispositif et des moyens de connexion électrique d'un autre élément, par exemple la protubérance d'une fiche.

Le dispositif peut comprendre un empilement de deux couches avec une première couche comprenant lesdits moyens de connexion électrique et une deuxième couche comprenant lesdits composants, montés sur un circuit flexible.

Le dispositif peut alors avantageusement comprendre une troisième couche flexible, en contact avec ladite deuxième couche, pour assurer son encapsulation.

Dans une première variante, lesdits moyens de connexion électrique sont avantageusement réalisés en un matériau métallique et notamment un métal ferromagnétique.

Dans une deuxième variante, lesdits moyens de connexion électrique comprennent deux pièces assemblées sur un support en un matériau polymère, l'une remplissant une fonction électrique et l'autre, une fonction magnétique.

Lorsque deux moyens de connexion électrique sont situés dans deux zones rigides adjacentes et séparés par une zone flexible de largeur L, ils présentent, de préférence, une hauteur H qui satisfait à l'inégalité L < π H et, de préférence à l'inégalité L < 2 H.

Grâce à cette disposition, les moyens de connexion électrique remplissent également une fonction de limitation de la flexion du dispositif dans les zones flexibles.

Le dispositif selon l'invention comprend avantageusement au moins un élément rigide complémentaire disposé sur la face du dispositif opposée auxdits moyens de connexion électrique et aligné, dans au moins une zone rigide, avec lesdits composants.

Cet élément rigide complémentaire contribue à la protection mécanique des composants du dispositif.

L'invention concerne également un ensemble comportant un dispositif selon l'invention et au moins une fiche reliée à un conducteur électrique, ladite fiche comportant des moyens de connexion électrique susceptibles de coopérer avec les moyens de connexion électrique dudit dispositif.

Ladite fiche comporte une pièce remplissant avantageusement une fonction magnétique.

L'invention sera mieux comprise et d'autres buts, avantages et caractéristiques de celle-ci apparaîtront plus clairement à la lecture de la description qui suit et qui est faite au regard des dessins annexés, sur lesquels :
- la figure 1 (1A-1D) comprend une vue de dessus (figure 1A) de la couche inférieure d'un dispositif selon l'invention, une vue de dessus (figure 1B) et une vue de dessous (figure 1C) de la couche intermédiaire de ce dispositif et une vue de dessus (figure 1D) de la couche supérieure de ce dispositif,
- la figure 2 est une vue en coupe partielle de la couche supérieure illustrée à la figure 1D, assemblée sur la couche intermédiaire illustrée aux figures 1B et 1C,
- la figure 3 (3A-3C) comprend une vue de dessous (figure 3A) et une vue en coupe (figure 3B) selon la ligne II-II de la figure 3A d'une fiche adaptée au dispositif selon l'invention illustré aux figures 1 et 2, ainsi qu'une vue en coupe (figure 3C) représentant la fiche illustrée aux figures 3A et 3B connectée au dispositif illustré aux figures 1 et 2,
- la figure 4 est une schématique éclatée et en perspective du dispositif illustré aux figures 1 et 2,
- la figure 5 (figures 5A-5B) comprend une vue en perspective (figure 5A) et une vue de dessus (figure 5B) du dispositif selon l'invention, après assemblage,
- la figure 6 est une vue de dessus du dispositif selon l'invention illustré à la figure 5, connecté à deux électrodes cutanées, et
- la figure 7 (7A-7B) comprend une vue de dessus (figure 7A) et une vue en coupe (figure 7B) d'une variante des moyens de connexion électrique illustrés sur les figures 1D et 2.

Les éléments communs aux différentes figures seront illustrés par les mêmes références.

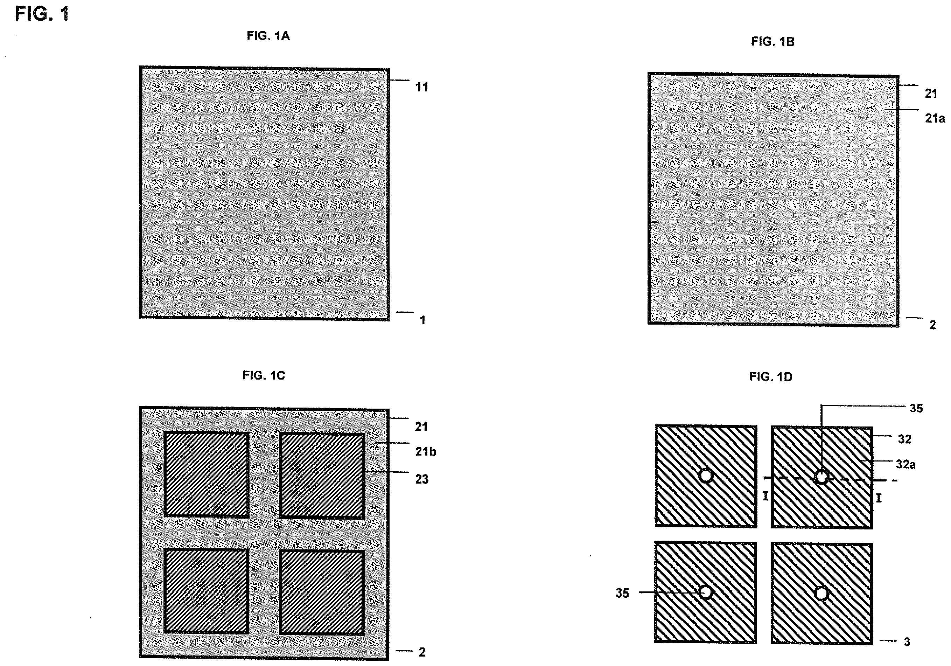

La figure 1 illustre schématiquement le dispositif selon l'invention, du type patch.

Celui-ci peut être décomposé en trois couches :
- une couche inférieure 1 qui comprend une feuille flexible jouant un rôle d'encapsulation inférieure,
- une couche intermédiaire 2 qui comprend des composants montés sur un circuit flexible, et
- une couche supérieure 3 qui comprend des éléments remplissant une double fonction de connexion électrique et de protection mécanique des composants.

La figure 1A est une vue de dessus représentant la couche inférieure 1 qui comprend une feuille flexible 11 jouant un rôle d'encapsulation inférieure.

La couche inférieure 1 est destinée à être en contact avec la peau de l'utilisateur.

La feuille flexible 11 est par exemple une feuille en élastomère thermoplastique ou en silicone, d'épaisseur comprise entre 0,1 mm et 1 mm.

Dans l'exemple illustré sur la figure 1A, la couche 1 présente une forme carrée. D'autres formes sont envisageables, notamment une forme rectangulaire, circulaire ou ovale.

Les figures 1B et 1C représentent la couche intermédiaire 2, respectivement vue de dessus et de dessous. Elle comprend un circuit flexible 21 dont la face supérieure 21a est représentée à la figure 1B et dont la face inférieure 21b est représentée à la figure 1C.

Dans l'exemple illustré, le circuit 21 présente une forme carrée. D'autres formes sont envisageables, notamment une forme rectangulaire, circulaire ou ovale.

Le circuit flexible 21 est par exemple supporté par une feuille en polyimide d'épaisseur 0,045 mm.

Des pistes métalliques (non représentées sur les figures) sont définies sur la face supérieure 21a du circuit flexible 21.

Les pistes métalliques sont par exemple des pistes en cuivre dont l'épaisseur est d'environ 0,035 mm.

Des ensembles 23 de composants sont montés sur la face inférieure 21b du circuit flexible 21. Dans l'exemple illustré, chaque ensemble 23 présente une forme carrée et quatre ensembles sont présents sur la face inférieure 21b.

Chaque ensemble 23 peut comprendre des composants électroniques, par exemple des circuits intégrés, des résistances, des inductances, des condensateurs ou des diodes, des sources d'énergie, par exemple des piles, des accumulateurs, des batteries ou des super-condensateurs ou tout autre composant.

De façon générale, tous ces composants sont susceptibles d'être détériorés s'ils subissent une contrainte.

L'épaisseur maximale des composants au sein de chaque ensemble 23 est typiquement comprise entre 0,1 mm et 5 mm. Cette gamme d'épaisseur est essentiellement fixée par les épaisseurs des sources d'énergie qui peuvent être intégrées dans un patch générateur d'impulsions pour l'électrostimulation, depuis des batteries en couches minces d'épaisseur de l'ordre de quelques centaines de microns, jusqu'à des batteries dans des packagings du type « *bouton* » ou « *pouch* » dont l'épaisseur est de l'ordre de quelques millimètres. Des pistes métalliques (non représentées sur les figures) sont définies sur la face inférieure 21b du circuit flexible 21 pour interconnecter les composants.

Des interconnexions verticales (non représentées sur les figures) permettent de connecter les composants situés sur la face inférieure 21b avec les pistes métalliques situées sur la face supérieure 21a.

La figure 1D est une vue de dessus représentant la couche supérieure 3, qui comprend des éléments 32.

Chaque élément 32 joue un double rôle de connexion électrique et de protection mécanique, comme cela va maintenant être expliqué en détail.

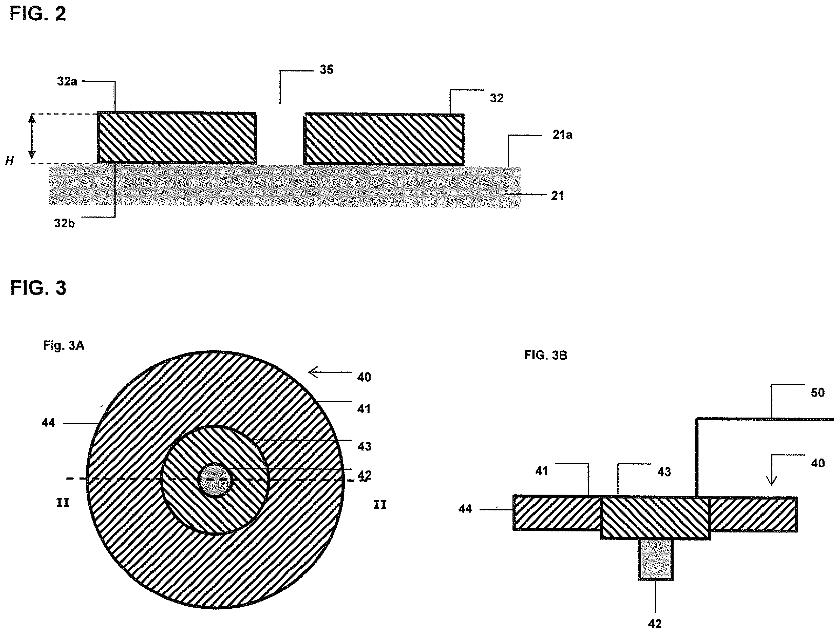

Il est fait référence à la figure 2 qui est une vue en coupe selon la ligne I-I sur la figure 1D d'un élément 32 assemblé sur le circuit flexible 21 illustré aux figures 1B et 1C.

L'élément 32 présente une épaisseur notée H et il est délimité par une face supérieure 32a et une face inférieure 32b par laquelle il est assemblé sur la face supérieure 21a du circuit flexible 21.

Plus précisément, la face inférieure 32b de l'élément 32 est brasée à des pistes métalliques définies sur la face supérieure 21a du circuit flexible 21 (non représentées sur les figures). Ainsi, l'élément 32 est relié électriquement au circuit flexible 21.

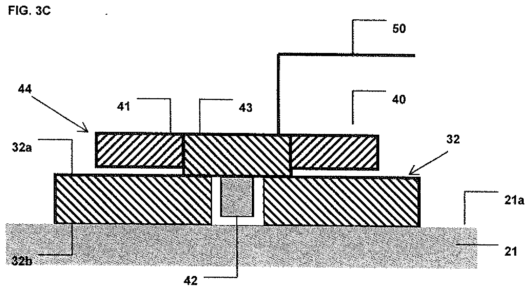

Comme cela sera décrit plus précisément au regard de la figure 3, l'élément 32 peut ainsi remplir une fonction de connexion électrique.

Pour cela, l'élément 32 est conçu pour coopérer avec le moyen de connexion d'une fiche destinée à être solidaire d'un conducteur électrique.

Dans l'exemple illustré aux figures 1D et 2, l'élément 32 est percé sur toute son épaisseur d'un trou 35. Il s'agit donc d'un trou traversant.

Ce trou peut notamment être cylindrique. Dans ce cas, son diamètre est avantageusement compris entre 0,1 mm et 10 mm et de préférence, entre 0,5 mm et 5 mm.

A titre de variante, une pluralité de trous traversants pourrait être prévue dans l'élément 32. Ceci permet de faciliter la connexion entre l'élément 32 et la fiche 40 décrite en référence à la figure 3.

Par ailleurs, l'élément 32 peut non seulement remplir une fonction électrique mais également une fonction magnétique. L'intérêt de cette fonction supplémentaire sera décrit en référence à la figure 3.

Dans ce cas, l'élément 32 est par exemple réalisé en un matériau ferromagnétique, tel qu'un acier ferromagnétique.

Les figures 3A et 3B représentent une fiche 40, respectivement vue de dessous et vue en coupe selon la ligne II-II sur la figure 3A.

La fiche 40 est solidaire d'un câble électrique 50, lui-même relié à une électrode cutanée qui n'est pas représentée sur la figure 3B.

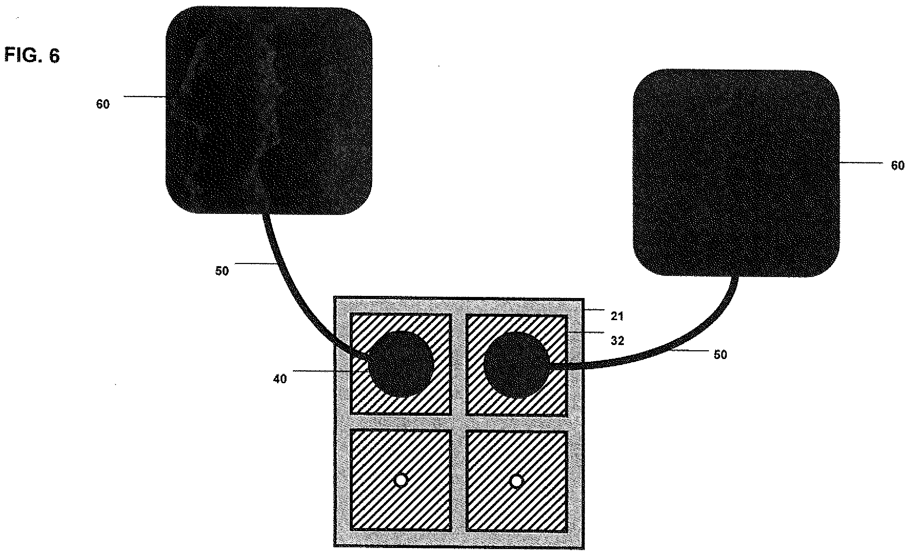

La fiche 40 est conçue pour coopérer avec un élément 32 afin d'établir une connexion électrique entre le dispositif et une électrode cutanée, comme cela sera décrit en référence à la figure 6.

La fiche 40 comprend une pièce plane 44, ici de forme cylindrique, formant la base de la fiche et une pièce 42 en saillie sur cette pièce plane formant une protubérance.

La pièce 44 comprend, dans cet exemple, une pièce annulaire 41 et une pièce cylindrique 43 au centre de la pièce annulaire 41. Les trois pièces 41, 43 et 42 sont centrées autour d'un même axe.

Dans cet exemple, la pièce 41 remplit une fonction magnétique.

Il s'agit par exemple d'une pièce en ferrite, aluminium-nickel-cobalt, samarium-cobalt ou encore en néodyme-fer-bore qui sont des matériaux aimantés.

La pièce 43 remplit une fonction électrique. Il s'agit par exemple d'une pièce en métal.

La pièce 42 est par exemple une pièce en polymère.

D'autres formes de réalisation de la fiche 40 pourraient être envisagées.

En particulier, la base 44 de la fiche pourrait être réalisée en une seule pièce, par exemple une pièce en un matériau aimanté et conducteur de l'électricité, notamment en aluminium-nickel-cobalt, samarium-cobalt ou néodyme-fer-bore. Dans ce cas, la protubérance 42 sera par exemple une pièce en métal.

Par ailleurs, dans l'exemple illustré à la figure 3B, la pièce annulaire 41 de la base 44 est légèrement en retrait par rapport à la pièce centrale cylindrique 43.

Cependant, dans un autre mode de réalisation, ces deux pièces 41 et 43 pourraient comporter la même épaisseur et être alignés.

Il est maintenant fait référence à la figure 3C qui représente la fiche 40 une fois connectée à un élément 32.

Le trou 35 accueille la protubérance 42, ce qui empêche une déconnexion accidentelle due à des forces latérales, c'est-à-dire des forces dans le plan de l'élément 32.

La face supérieure 32a de l'élément 32 établit un contact électrique avec la pièce centrale 43. L'élément 32 et la pièce 41 exercent l'un sur l'autre une force magnétique attractive.

Dans l'exemple de réalisation de la fiche 40 illustré à la figure 3, le contact électrique entre la face supérieure 32a et la pièce 43 est amélioré, par rapport à un mode de réalisation où la pièce annulaire 41 et la pièce centrale 43 présentent la même épaisseur.

En effet, du fait que la pièce 41 est légèrement en retrait de la pièce 43, la pression exercée par la pièce 43 sur la couche supérieure 32a est augmentée. En d'autres termes, la résistance électrique du contact est diminuée.

Lorsque la base 44 de la fiche est réalisée en une seule pièce, le contact électrique entre la fiche et l'élément 32 est réalisée entre la face supérieure 32a de l'élément 32 et toute la surface de la base en contact avec cette face supérieure 32a.

La présence de pièces remplissant une fonction magnétique permet de faciliter la connexion entre la fiche 44 et un élément 32. En effet, un petit déplacement latéral de la fiche suffit pour que les forces magnétiques entrent en action et assemblent la fiche 44 et l'élément 32, en provoquant l'insertion de la protubérance 42 de la fiche dans le trou 35 de l'élément 32.

Cependant, il n'est pas indispensable de prévoir des pièces remplissant une fonction magnétique. En effet, comme cela apparaîtra aux figures suivantes, la présence de plusieurs éléments 32 sur le dispositif selon l'invention facilite déjà une connexion sans contrôle visuel, entre le dispositif et une fiche reliée par exemple à une électrode cutanée.

L'épaisseur H de chaque élément 32 est choisie pour être suffisamment élevée de façon à assurer le maintien latéral de la pièce 42 et donc de la fiche 40 dans l'élément 32.

L'épaisseur H de chaque élément 32 est, de préférence, comprise entre 0,5 mm et 2 mm.

La surface de chaque élément 32 et la surface de la pièce annulaire 41 sont suffisamment grandes pour assurer, grâce à l'attraction magnétique, le maintien vertical de la pièce annulaire 41 et donc de la fiche 40.

La surface de chaque élément 32 est de préférence comprise entre 1 cm² et 10 cm².

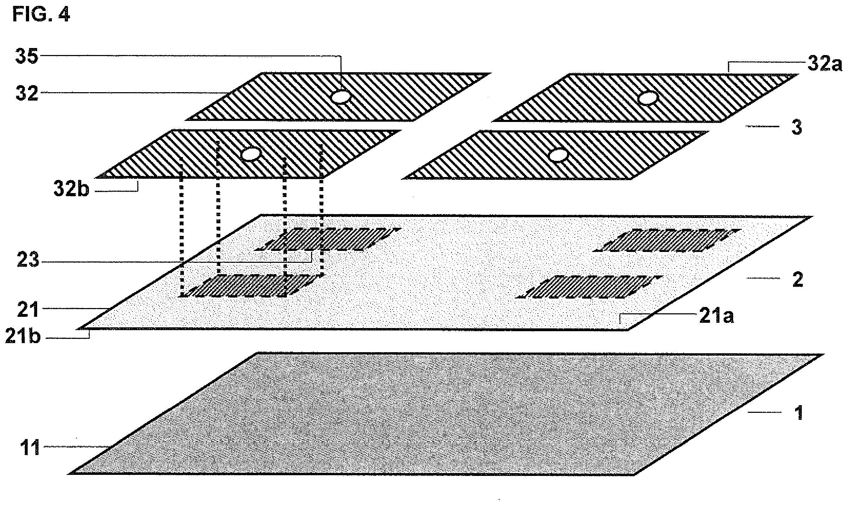

La figure 4 représente schématiquement une vue éclatée et en perspective du dispositif illustré aux figures 1 et 2.

Elle montre comment les éléments 32 remplissent leur fonction de protection mécanique.

Ainsi, la figure 4 illustre les trois couches 1, 2 et 3 du dispositif selon l'invention qui sont disposées successivement les unes au dessus des autres.

La couche supérieure 3 se trouve ainsi en regard de la face supérieure 21a de la couche intermédiaire 2 et les ensembles de composants 23 de la couche intermédiaire 2 sont en regard de la couche inférieure 1.

La figure 4 montre que les ensembles 23 de composants de la couche intermédiaire 2 ainsi que les éléments 32 de la couche supérieure 3 sont localisés, respectivement dans la couche intermédiaire 2 et dans la couche supérieure 3, de façon à ce qu'un élément 32 de la couche supérieure 3 recouvre un ensemble de composants 23.

En pratique, ceci peut être obtenu avec des éléments 32 ayant une surface de projection sur la couche intermédiaire 2 qui est au moins égale à la surface de l'ensemble 23 de composants auquel il est associé et en disposant chaque élément 32 dans la couche supérieure 3 de façon à ce qu'il soit aligné verticalement avec un ensemble 23 de composants. En d'autres termes, un élément 32 est aligné avec un ensemble de composants selon l'épaisseur de l'empilement des trois couches 1, 2 et 3.

Dans ce cas, chaque élément 32 est bien susceptible de recouvrir un ensemble 23 de composants de la couche intermédiaire 2.

Grâce à cette disposition, les éléments 32 assurent une protection mécanique efficace des composants situés dans les ensembles 23, notamment contre les chocs, le percement ou les contraintes lors de la mise en flexion du patch. Ceci est permis par :
- leur épaisseur, qui peut par exemple être supérieure à 0,5 mm,
- leur composition, les éléments 32 pouvant par exemple être réalisés en acier, qui est un matériau rigide et tenace.

L'invention n'est pas cependant limitée à ce mode de réalisation. En pratique, les moyens de connexion ne sont pas nécessairement conçus pour protéger tous les composants présents sur la couche intermédiaire. En effet, certains composants moins sensibles ou moins fragiles peuvent ne pas être protégés. Il suffit donc que les moyens de connexion électrique recouvrent et protègent au moins un composant. Ainsi, il suffit que les moyens de connexion soient alignés avec au moins un composant, selon l'épaisseur de l'empilement.

Par ailleurs, dans les exemples illustrés précédemment, les composants sont disposés sur la face inférieure 21b de la couche intermédiaire 2.

Cependant, l'invention concerne également un dispositif dans lequel les composants sont disposés sur la face supérieure 21a de la couche intermédiaire. Dans ce cas, les moyens de connexion électrique sont conçus pour présenter la forme d'un couvercle.

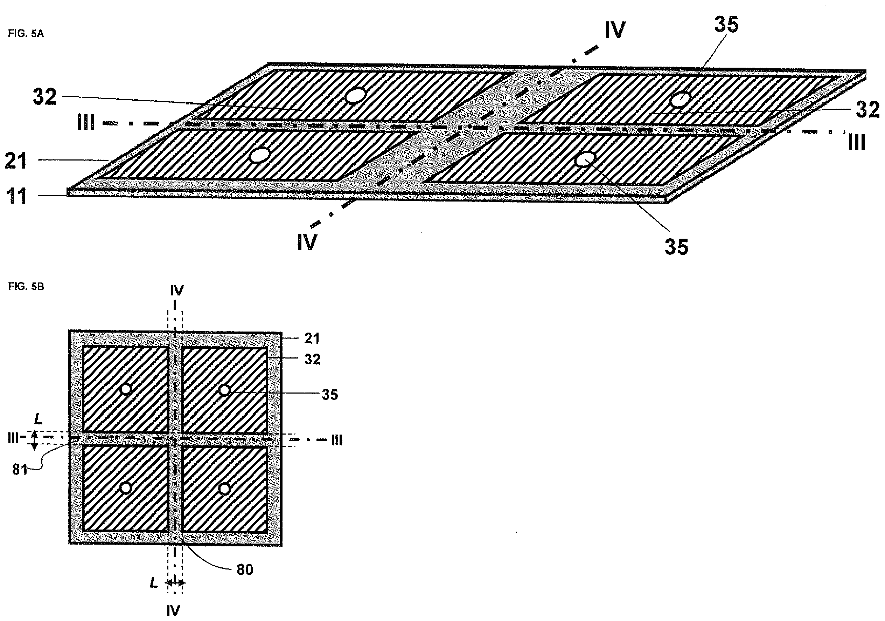

Les figures 5A et 5B représentent schématiquement le dispositif illustré à la figure 4 une fois assemblé, respectivement vu en perspective et vu de dessus.

Les figures 5A et 5B confirment que chaque ensemble de composants 23 présent sur la couche intermédiaire 2 est recouvert par un élément 32.

Par ailleurs, compte tenu des gammes d'épaisseur indiquées précédemment pour les différents éléments constitutifs du dispositif, l'épaisseur maximale du dispositif obtenu est comprise entre 1 mm et 8 mm.

De plus, la surface totale du patch est comprise entre 5 cm² et 50 cm².

Ainsi, le dispositif comprend une pluralité de zones rigides, correspondant aux zones occupées par les éléments 32, reliées par des zones flexibles, correspondant au reste de la surface du dispositif.

Dans l'exemple illustré à la figure 5, ces zones flexibles sont deux bandes 80 et 81 du circuit flexible 21 de la couche intermédiaire 2. Ces zones flexibles 80 et 81 forment ici une croix entre les quatre éléments 32 du dispositif illustré à la figure 5.

La largeur L de ces zones 80 et 81 est, de préférence, comprise entre 1 mm et 5 cm.

Bien entendu, ces zones flexibles peuvent présenter des formes différentes, suivant la forme des éléments 32 et leur répartition sur le circuit flexible 21.

Dans l'exemple illustré à la figure 5, le dispositif selon l'invention peut être fléchi selon les axes III-III et IV-IV passant dans les zones flexibles 80 et 81 et représentés en pointillés sur la figure 5.

Grâce à la présence de ces zones flexibles, le dispositif selon l'invention peut être adapté à des zones différentes du corps humain, du fait de sa capacité de conformation.

Par ailleurs, les éléments 32 peuvent remplir une troisième fonction, en plus de leur fonction de connexion électrique et de leur fonction de protection mécanique des composants. Cette fonction consiste à limiter la flexion du dispositif, c'est-à-dire à limiter le rayon de courbure au niveau des zones flexibles 80 et 81 à une valeur déterminée, ce qui permet notamment de protéger les pistes métalliques du circuit 21 présentes au niveau des zones flexibles 80 et 81.

Les éléments 32 peuvent remplir cette troisième fonction de limitation du rayon de courbure de la manière suivante : lors d'une mise en flexion au niveau d'une zone flexible 80 ou 81, deux éléments 32 adjacents entrent en contact l'un avec l'autre, limitant ainsi le rayon de courbure à une valeur déterminée. La valeur minimale du rayon de courbure dépend de largeur L des zones flexibles 80 et 81 et de la hauteur H des éléments 32.

Afin d'obtenir cet effet de limitation du rayon de courbure, il convient que L et H soient choisis de telle sorte que L < π H et, de préférence, L < 2 H.

Ainsi, le dispositif selon l'invention comprend une pluralité de moyens de connexion constitués par les éléments 32, chacun d'eux pouvant coopérer avec une fiche 40 pour assurer la connexion entre le dispositif et une électrode cutanée par exemple.

La figure 6 représente schématiquement un dispositif selon l'invention connecté à deux électrodes cutanées 60.

Chaque électrode cutanée 60 est reliée à un câble électrique 50 solidaire d'une fiche 40 qui coopère avec un élément 32 du dispositif pour établir une connexion électrique entre le dispositif et l'électrode cutanée 60.

Ainsi, dans l'exemple illustré à la figure 6, le dispositif est relié à deux électrodes cutanées 60, typiquement une cathode et une anode et comprend quatre moyens de connexion électrique 32.

Le fait de disposer d'un nombre de moyens de connexion 32 (quatre dans cet exemple) supérieur au nombre de fiches 40 à connecter (deux dans cet exemple) est avantageux, car cela offre à l'utilisateur une grande liberté de positionnement des électrodes cutanées sur le corps, pour une position donnée du dispositif.

Ceci facilite également la réalisation de la connexion puisque plusieurs possibilités de connexion sont offertes à chaque fiche.

Dans une variante du dispositif décrit aux figures 1 à 6, des éléments rigides peuvent être ajoutés en face arrière du dispositif, par exemple sur la face de la feuille flexible 11 opposée à la couche intermédiaire 2.

Chacun de ces éléments rigides supplémentaires (non représentés sur les figures) est aligné verticalement avec un ensemble 23 de composants. Ainsi, ces éléments rigides supplémentaires contribuent à la protection mécanique des composants situés dans les ensembles 23.

De plus, ces éléments rigides supplémentaires peuvent assurer une fonction de limitation du rayon de courbure, de la même manière que les éléments 32.

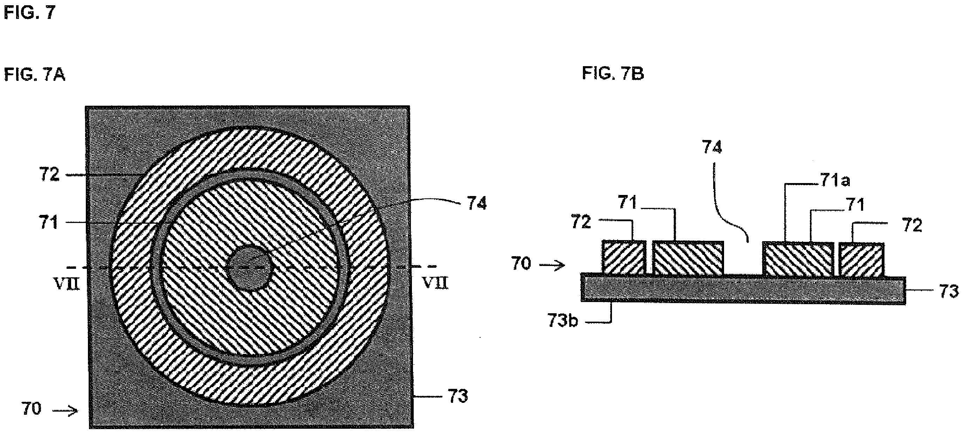

Dans une variante du dispositif décrit aux figures 1 à 6, les éléments 32 peuvent être remplacés par d'autres types d'éléments, remplissant également un double rôle de connexion électrique et de protection mécanique. Par exemple, chaque élément 32 peut être remplacé par un élément 70 décrit à la figure 7.

L'élément 70 comprend une pièce annulaire centrale 71 remplissant une fonction électrique. La pièce 71 est par exemple une pièce en métal.

L'élément 70 comprend également une pièce annulaire 72 entourant la pièce annulaire 71 et remplissant une fonction magnétique. La pièce 72 est formée dans un matériau aimanté, par exemple ferrite, aluminium-nickel-cobalt, samarium-cobalt ou néodyme-fer-bore.

Les deux pièces annulaires 71 et 72 sont centrées autour d'un même axe, et définissent en leur centre un trou 74.

L'élément 70 comprend enfin une pièce plane 73, formée dans un matériau rigide, par exemple un matériau polymère comme une résine époxy. La pièce 73 remplit une fonction mécanique, en maintenant les deux pièces 71 et 72 dans un même plan.

La face inférieure 73b de la pièce 73 est destinée à être collée sur la face supérieure 21a du circuit flexible 21. Des interconnexions verticales réalisées dans la pièce 73 et dans le circuit flexible 21 (non représentées sur les figures) permettent de relier électriquement la pièce 71 aux pistes métalliques du circuit 21.

Chaque élément 70 remplit un rôle de connexion électrique. Ainsi, une fiche 40 peut coopérer avec un élément 70 afin d'établir une connexion électrique entre le dispositif et une électrode cutanée.

Le trou 74 de l'élément 70 accueille alors la protubérance 42 de la fiche 40, ce qui empêche une déconnexion accidentelle due à des forces latérales, c'est-à-dire des forces dans le plan de la pièce 73.

La face supérieure 71a de la pièce 71 établit un contact électrique avec la pièce centrale 43 de la fiche 40.

La pièce 72 de l'élément 70 et la pièce 41 de la fiche 40 exercent l'une sur l'autre une force magnétique attractive.

Comme mentionné précédemment, la pièce 73 de l'élément 70 maintient les deux pièces 71 et 72 dans un même plan, ce qui assure :
- un bon contact électrique entre la pièce 71 et la pièce 43 quand la pièce 72 et la pièce 41 exercent l'une sur l'autre une force magnétique attractive ;
- une attraction magnétique maximale entre la pièce 72 et la pièce 41 quand la pièce 71 et la pièce 43 sont en contact électrique.

Le principal avantage d'un élément 70 comparé à un élément 32 est le suivant. Dans le cas où l'utilisateur cherche à connecter une fiche 40 dans un élément 70, l'attraction magnétique s'exerce entre deux pièces annulaires aimantées (la pièce 72 de l'élément 70 et la pièce 41 de la fiche 40), ce qui permet de guider très efficacement la protubérance 42 de la fiche 40 dans le trou 74 de l'élément 70, car deux aimants annulaires ont tendance à s'aligner, c'est-à-dire à se centrer autour d'un même axe.

En comparaison, dans le cas où l'utilisateur cherche à connecter une fiche 40 dans un élément 32, l'attraction magnétique s'exerce entre une pièce annulaire aimantée (la pièce 41 de la fiche 40) et une pièce formée dans un métal ferromagnétique (la pièce 32), ce qui peut conduire à des situations où la fiche et l'élément 32 ne sont pas alignés correctement et donc où il est plus difficile de faire pénétrer la protubérance 42 dans le trou 35.

Cet inconvénient peut être surmonté en prévoyant une pluralité de trous traversants dans les éléments 32.

Comme les éléments 32, chaque élément 70 remplit également un rôle de protection mécanique des composants situés dans les ensembles 23, notamment contre les chocs, le percement ou les contraintes lors de la mise en flexion du patch.

Ceci est permis par :
- l'épaisseur des éléments 70, qui peut par exemple être supérieure à 0,5 mm,
- la composition des éléments 70, qui sont réalisés en assemblant des matériaux rigides et tenaces, par exemple un métal (pièce 71) et une résine époxy (pièce 73).

Enfin, comme cela a été décrit pour les éléments 32 en référence à la figure 5, les éléments 70 peuvent également remplir une fonction de limitation de la flexion au niveau des zones flexibles situées entre deux éléments adjacents.

Les signes de référence insérés après les caractéristiques techniques figurant dans les revendications ont pour seul but de faciliter la compréhension de ces dernières et ne sauraient en limiter la portée.

## Revendications

1. Dispositif configuré pour être fixé sur la peau d'un utilisateur, sensiblement plan et comprenant des composants ainsi que des moyens de connexion électrique définissant au moins une zone rigide du dispositif, les moyens de connexion électrique (32, 70) présentant, dans le plan du dispositif, une surface correspondant à au moins un composant et étant disposés de manière à recouvrir ledit au moins un composant pour assurer sa protection mécanique, **caractérisé en ce que** ledit dispositif comprend en outre un empilement de deux couches avec une première couche (3) comprenant lesdits moyens de connexion électrique (32, 70) et une deuxième couche (2) comprenant lesdits composants (23), montés sur un circuit flexible.

2. Dispositif selon la revendication 1, dans lequel les moyens de connexion électrique (32, 70) définissent au moins deux zones rigides reliées électriquement et mécaniquement par une zone flexible, lesdites zones rigides portant des composants.

3. Dispositif selon la revendication 1 ou 2, dans lequel les moyens de connexion électrique (32, 70) présentent, dans le plan du dispositif, une surface supérieure à celle occupée par lesdits composants et sont alignés avec lesdits composants selon l'épaisseur d'au moins une zone rigide, de manière à les recouvrir.

4. Dispositif selon l'une des revendications 1 à 3, dans lequel lesdits moyens de connexion électrique (32, 70) comportent au moins un trou (35, 74) destiné à coopérer avec une protubérance d'une fiche reliée à un conducteur électrique.

5. Dispositif selon l'une des revendications 1 à 4, dans lequel lesdits moyens de connexion électrique (32, 70) remplissent aussi une fonction magnétique.

6. Dispositif selon l'une des revendications 1 à 5, comprenant une troisième couche (1) flexible, en contact avec ladite deuxième couche (2), pour assurer son encapsulation.

7. Dispositif selon l'une des revendications 1 à 6, dans lequel lesdits moyens de connexion électrique (32) sont réalisés en un matériau métallique et notamment un métal ferromagnétique.

8. Dispositif selon l'une des revendications 1 à 6, dans lequel lesdits moyens de connexion électrique (70) comprennent deux pièces assemblées sur un support en un matériau polymère, l'une (71) remplissant une fonction électrique et l'autre (72) une fonction magnétique.

9. Dispositif selon l'une des revendications 2 à 6, dans lequel deux moyens de connexion électrique (32) situés dans deux zones rigides adjacentes et séparés par une zone flexible de largeur L, présentent une hauteur H qui satisfait à l'inégalité L < π H et, de préférence à l'inégalité L < 2 H.

10. Dispositif selon l'une des revendications 1 à 9, comprenant au moins un élément rigide complémentaire disposé sur la face du dispositif opposée auxdits moyens de connexion électrique et aligné, dans au moins une zone rigide, avec lesdits composants.

11. Ensemble comportant un dispositif selon l'une des revendications 1 à 10 et au moins une fiche (40) reliée à un conducteur électrique (50), ladite fiche comportant des moyens de connexion électrique (43) susceptibles de coopérer avec les moyens de connexion électrique (32) dudit dispositif.

12. Ensemble selon la revendication 11, dans lequel ladite fiche (40) comporte une pièce (41) remplissant une fonction magnétique.

## Patentansprüche

1. Vorrichtung, die konfiguriert ist, um auf der Haut eines Benutzers, die im Wesentlichen eben ist, befestigt zu werden, und Bauteile sowie elektrische Verbindungsmittel umfasst, die mindestens eine starre Zone der Vorrichtung definieren, wobei die elektrischen Verbindungsmittel (32, 70) in der Ebene der Vorrichtung eine Oberfläche aufweisen, die mindestens einem Bauteil entspricht und derart angeordnet ist, um das mindestens eine Bauteil zu bedecken, um seinen mechanischen Schutz sicherzustellen, **dadurch gekennzeichnet, dass** die Vorrichtung außerdem eine Stapelung aus zwei Schichten umfasst, mit einer ersten Schicht (3), die die elektrischen Verbindungsmittel (32, 70) umfasst, und einer zweiten Schicht (2), die die Bauteile (23), die auf einer biegsamen Schaltung montiert sind, umfasst.

2. Vorrichtung nach Anspruch 1, wobei die elektrischen Verbindungsmittel (32, 70) mindestens zwei starre Zonen definieren, die elektrisch und mechanisch durch eine biegsame Zone verbunden sind, wobei die starren Zonen Bauteile tragen.

3. Vorrichtung nach Anspruch 1 oder 2, wobei die elektrischen Verbindungsmittel (32, 70) in der Ebene der Vorrichtung eine Oberfläche aufweisen, die größer ist als die, die von den Bauteilen belegt wird, und die mit den Bauteilen gemäß der Stärke mindestens einer starren Zone derart ausgerichtet sind, um sie zu bedecken.

4. Vorrichtung nach einem der Ansprüche 1 bis 3, wobei die elektrischen Verbindungsmittel (32, 70) mindestens ein Loch (35, 74) beinhalten, das dazu bestimmt ist, mit einem Vorsprung eines Steckverbinders, der mit einem elektrischen Leiter verbunden ist, zusammenzuwirken.

5. Vorrichtung nach einem der Ansprüche 1 bis 4, wobei die elektrischen Verbindungsmittel (32, 70) auch eine magnetische Funktion erfüllen.

6. Vorrichtung nach einem der Ansprüche 1 bis 5, die eine biegsame dritte Schicht (1) in Kontakt mit der zweiten Schicht (2) umfasst, um ihre Kapselung sicherzustellen.

7. Vorrichtung nach einem der Ansprüche 1 bis 6, wobei die elektrischen Verbindungsmittel (32) aus einem metallischen Material und insbesondere einem ferromagnetischen Metall realisiert sind.

8. Vorrichtung nach einem der Ansprüche 1 bis 6, wobei die elektrischen Verbindungsmittel (70) zwei Teile umfassen, die auf einem Träger aus einem Polymermaterial zusammengefügt sind, wobei das eine (71) eine elektrische Funktion und das andere (72) eine magnetische Funktion erfüllt.

9. Vorrichtung nach einem der Ansprüche 2 bis 6, wobei zwei elektrische Verbindungsmittel (32), die in zwei aneinandergrenzenden starren Zonen liegen und durch eine biegsame Zone der Breite L getrennt sind, eine Höhe H aufweisen, die die Ungleichheit L < π H und bevorzugt die Ungleichheit L < 2 H erfüllt.

10. Vorrichtung nach einem der Ansprüche 1 bis 9, die mindestens ein ergänzendes starres Element umfasst, das auf der Fläche der Vorrichtung, die den elektrischen Verbindungsmitteln gegenüberliegt, angeordnet ist, und in mindestens einer starren Zone mit den Bauteilen ausgerichtet ist.

11. Zusammenstellung, die eine Vorrichtung nach einem der Ansprüche 1 bis 10 und mindestens einen Steckverbinder (40), der mit einem elektrischen Leiter (50) verbunden ist, beinhaltet, wobei der Steckverbinder elektrische Verbindungsmittel (43) beinhaltet, die geeignet sind, um mit den elektrischen Verbindungsmitteln (32) der Vorrichtung zusammenzuwirken.

12. Zusammenstellung nach Anspruch 11, wobei der Steckverbinder (40) ein Teil (41) beinhaltet, das eine magnetische Funktion erfüllt.

## Claims

1. Device configured to be fixed on the skin of a user, substantially flat and comprising components as well as electrical connection means defining at least one rigid zone of the device, the electrical connection means (32, 70) having, in the plane of the device, a surface corresponding to at least one component and being arranged so as to cover said at least one component to ensure the mechanical protection thereof, **characterised in that** said device further comprises a stack of two layers with a first layer (3) comprising said electrical connection means (32, 70) and a second layer (2) comprising said components (23), mounted on a flexible circuit.

2. Device according to claim 1, wherein the electrical connection means (32, 70) defining at least two rigid zones connected electrically and mechanically by a flexible zone, said rigid zones carrying components.

3. Device according to claim 1 or 2, wherein the electrical connection means (32, 70) have, in the plane of the device, a surface area greater than that occupied by said components and are aligned with said components according to the thickness of at least one rigid zone, so as to cover them.

4. Device according to one of claims 1 to 3, wherein said electrical connection means (32, 70) comprise at least one hole (35, 74) intended to engage with a protrusion of a plug connected to an electrical conductor.

5. Device according to one of claims 1 to 4, wherein said electrical connection means (32, 70) also fulfil a magnetic function.

6. Device according to one of claims 1 to 5, comprising a third flexible layer (1), in contact with said second layer (2), to ensure the encapsulation thereof.

7. Device according to one of claims 1 to 6, wherein said electrical connection means (32) are made of a metal material and in particular, a ferromagnetic material.

8. Device according to one of claims 1 to 6, wherein said electrical connection means (70) comprise two parts assembled on a support made of a polymer material, one (71) fulfilling an electrical function and the other (72) fulfilling a magnetic function.

9. Device according to one of claims 2 to 6, wherein two electrical connection means (32) situated in two adjacent rigid zones and separated by a flexible zone of width L, have a height H which satisfies the inequality L < π H and, preferably the inequality L < 2 H.

10. Device according to one of claims 1 to 9, comprising at least one complementary rigid element arranged on the face of the device opposite said electrical connection means and aligned, in at least one rigid zone, with said components.

11. Assembly comprising a device according to one of claims 1 to 10 and at least one plug (40) connected to an electrical conductor (50), said plug comprising electrical connection means (43) capable of engaging with the electrical connection means (32) of said device.

12. Assembly according to claim 11, wherein said plug (40) comprises a part (41) fulfilling a magnetic function.
